(19) Europäisches Patentamt
European Patent Office
Office européen des brevets

(11) EP 0 728 736 B1

(12) EUROPÄISCHE PATENTSCHRIFT

(45) Veröffentlichungstag und Bekanntmachung des Hinweises auf die Patenterteilung:
**19.08.1998 Patentblatt 1998/34**

(51) Int Cl.⁶: **C07C 215/08**, C07B 57/00, C07C 233/83

(21) Anmeldenummer: **96101955.1**

(22) Anmeldetag: **10.02.1996**

# (54) Verfahren zur Herstellung von optisch aktivem tert-Leucinol und dessen Verwendung

Process for the preparation of optically active tert-leucinol and use thereof

Procédé pour la préparation de tert-leucinol optiquement actif et son utilisation

(84) Benannte Vertragsstaaten:
**AT BE CH DE DK ES FR GB IE IT LI NL PT SE**

(30) Priorität: **21.02.1995 DE 19505994**

(43) Veröffentlichungstag der Anmeldung:
**28.08.1996 Patentblatt 1996/35**

(73) Patentinhaber: **Degussa Aktiengesellschaft
60311 Frankturt (DE)**

(72) Erfinder:
- **Drauz, Karlheinz, Prof. Dr.
D-63579 Freigericht (DE)**
- **Jahn, Wilfried
D-63571 Gelnhausen (DE)**
- **Schwarm, Michael, Dr.
D-63755 (DE)**

(56) Entgegenhaltungen:
**EP-A- 0 036 265 GB-A- 1 471 838**

- **CHEMISTRY A EUROPEAN JOURNAL, Bd. 1, Nr. 8, November 1995, D-WEINHEIM, Seiten 538-540, XP000195975 KARLHEINZ DRAUZ ET AL.: "Synthesis of (R)-tert-Leucinol by classical Resolution of the racemic mixture"**
- **JOURNAL OF ORGANIC CHEMISTRY, Bd. 58, Nr. 13, 18.Juni 1993, EASTON US, Seiten 3568-3571, XP002001481 MARC J. MCKENNON ET AL. : "A Convenient Reduction of Amino Acids and Their Derivatives"**
- **TETRAHEDRON LETTERS, Bd. 33, Nr. 38, 1992, OXFORD GB, Seiten 5517-5518, XP002001482 ATSUSHI ABIKO ET AL.: "An Improved, Convenient procedure for Reduction of Amino Acids to Aminoalcohols: Use of NaBH4 - H2SO4"**
- **ORGANOMETALLICS, Bd. 8, Nr. 1, Januar 1989, THE AMERICAN CHEMICAL SOCIETY, WASHINGTON, DC, USA, Seiten 846-850, XP000195926 HISAO NISHIYAMA ET AL.: "Chiral and C2-Symmetrical..."**
- **DATABASE WPI Week 8137 Derwent Publications Ltd., London, GB; AN 81-67110d XP002001483 & JP-A-56 095 149 (NISSHIN FLOUR MILL KK) , 1.August 1981**
- **J.MED.CHEM. 1992, 35, Seiten 2327-40**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 728 736 B1

Printed by Jouve, 75001 PARIS (FR)

**Beschreibung**

Die Erfindung betrifft ein Verfahren zur Herstellung von optisch aktivem tert-Leucinol (Formel I),

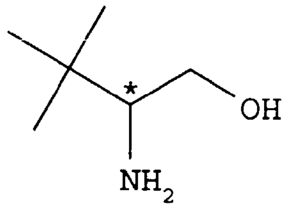

(I)

worin * ein Chiralitätszentrum bedeutet, bei dem racemisches (RS)-tert-Leucinol durch Umsetzung mit einer optisch aktiven Säure in ein diastereomeres Salzpaar überführt wird, dieses dann fraktioniert kristallisiert wird und daraus das optisch aktive tert-Leucinol durch Abtrennung der optisch aktiven Säure freigesetzt und ggf. in Substanz isoliert wird, und dessen Verwendung zur Herstellung von Derivaten.

Optisch aktives tert-Leucinol und seine Derivate finden verbreitete Anwendung in der asymmetrischen Synthese, wobei mit einer prochiralen Vorstufe Reaktionen durchgeführt werden, die in Gegenwart eines optisch aktiven tert-Leucinolderivates mit hoher Diastereo- oder Enantioselektivität ablaufen, da die sterisch besonders anspruchsvolle tert-Butylgruppe einen dirigierenden Einfluß auf die an den prochiralen Molekülen oder Molekülgruppen ablaufenden Reaktionen ausübt. Beispiele für solche Derivate, mit denen jeweils eine Vielzahl von asymmetrischen Reaktionen durchgeführt werden kann, sind 4-tert-Butyl-2-oxazolidinon (Formel II) (D. A. Evans, K. T. Chapman, J. Bisaha, J. Am. Chem. Soc. 1988, 110, 1238), Oxazoline (Formel III) (A. N. Hulme, A. I. Meyers, J. Org. Chem. 1994, 59, 952) und bicyclische Lactame (Formel IV) (D. Romo, A. I. Meyers, J. Org. Chem. 1992, 57, 6265)

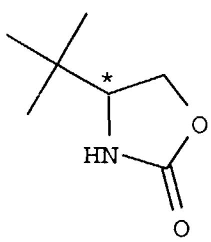

(II)

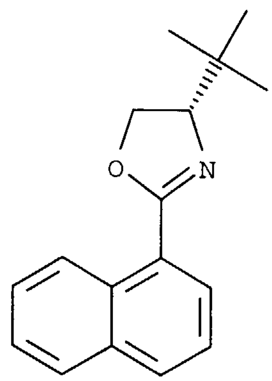

(III)

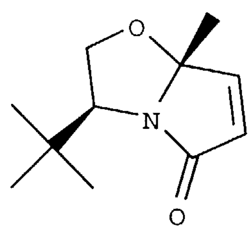

(IV)

oder Liganden zur Herstellung chiraler Katalysatoren wie Mercaptophenyloxazolin (Formel V) (Q.-L. Zhou, A. Pfaltz, Tetrahedron Lett. 1993, 34, 7725), Bisoxazolin (Formel VI) (D. A. Evans, S. J. Miller, T. Lectka, J. Am. Chem. Soc. 1993, 115, 6460) oder Bis(oxazolinyl)pyridin (Formel VII) (H. Nishiyama, H. Sakaguchi, T. Nakamura, M. Morihata, M. Kondo, K. Itoh, Organometallics 1989, 8, 846).

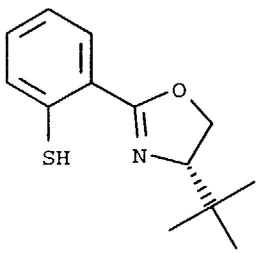 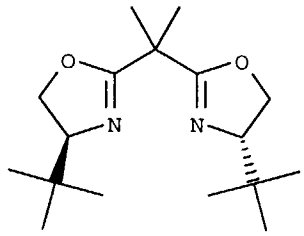 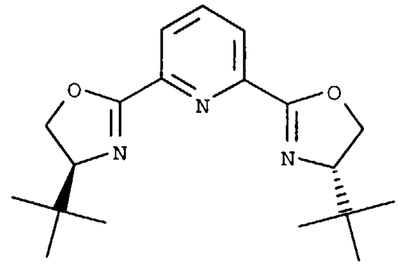

(V) (VI) (VII)

Ein Einbau eines optisch aktiven tert-Leucinols oder eines seiner Derivate in eine pharmazeutisch wirksame Substanz, die z. B. peptidartige Struktur haben könnte, ist ebenfalls denkbar.

Ein Problem, das den Einsatz von optisch aktivem tert-Leucinol für die oben geschilderten Zwecke unter anderem einschränkt, ist die bisher zum Teil schlechte Zugänglichkeit. Optisch aktives tert-Leucinol wurde bisher durch Reduktion des entsprechenden optisch aktiven tert-Leucins mit einem der für die Reduktion von Aminosäuren gängigen Reagentien hergestellt (H. Nishiyama, H. Sakaguchi, T. Nakamura, M. Morihata, M. Kondo, K. Itoh, Organometallics 1989, 8, 846; A. Abiko, S. Masamune, Tetrahedron Lett. 1992, 33, 5517; M. J. McKennon, A. I. Meyers, K. Drauz, M. Schwarm, J. Org. Chem. 1993, 58, 3568). Dies setzt jedoch voraus, daß das jeweilige tert-Leucin in optisch reiner Form verfügbar ist.

(S)-tert-Leucin ist enzymatisch über eine Transaminierung (EP 0 248 357 A2) oder insbesondere eine reduktive Aminierung von Trimethylbrenztraubensäure (A. S. Bommarius, K. Drauz, W. Hummel, M.-R. Kula, C. Wandrey, Biocatalysis 1994, 10, 37; U. Kragl, D. Vasic-Racki, C. Wandrey, Chem. Ing. Tech. 1992, 64, 499) in technischen Mengen problemlos zugänglich. (R)-tert-Leucin ist dagegen nur unter Schwierigkeiten über aufwendige chemische oder enzymatische Racematspaltungen zu erhalten (J. Viret, H. Patzelt, A. Collet, Tetrahedron Lett. 1986, 27, 5865 und dort zitierte Literatur), zumal auch die Herstellung des reinen racemischen (RS)-tert-Leucins mit relativ großem Aufwand verbunden ist (F. Knoop, G. Landmann, Z. Physiol. Chem. 1914, 89, 157).

Ferner ist es dem Fachmann grundsätzlich bekannt, racemische Verbindungen, sofern zugänglich, zu ihrer Spaltung in verschiedenen Lösungsmitteln mit optisch aktiven Säuren umzusetzen, um so diastereomere Salzpaare herzustellen und diese durch fraktionierte Kristallisation zu trennen. Auch wenn dieses Verfahren generell bekannt ist, so kann jedoch für den konkreten Fall nicht vorhergesagt werden, ob überhaupt und, wenn ja, mit welcher Säure ein kristallines Salz erhältlich ist und welches Enantiomere des racemischen Gemisches mit welchem Enantiomeren der Säure als Salz abgetrennt werden kann. Außerdem sollte die optisch aktive Säure aus Kostengründen leicht zugänglich und möglichst gut recyclierbar sein. Der zweite Punkt ist zusätzlich aus ökologischen Gründen von besonderer Bedeutung, da andernfalls eine erhebliche Menge Abfall entsteht.

Angesichts des hierin angegebenen und diskutierten Standes der Technik war es daher Aufgabe der Erfindung, ein Verfahren der eingangs erwähnten Gattung zur Verfügung zu stellen, das die Herstellung von optisch aktivem tert-Leucinol in hoher Ausbeute mit hoher optischer Reinheit hinsichtlich der Enantiomeren gestattet, ohne dafür optisch aktives tert-Leucin als Vorstufe reduzieren zu müssen, um insbesondere auch einen Zugang zu dem bisher nur schwer erhältlichen (R)-tert-Leucinol und seinen Derivaten zu eröffnen.

Gelöst werden diese sowie weitere nicht einzeln genannte Aufgaben durch ein Verfahren mit den Merkmalen des kennzeichnenden Teils des Anspruchs 1. Vorteilhafte Verfahrensmodifikationen sind Gegenstand der auf Anspruch 1 rückbezogenen abhängigen Verfahrensansprüche.

Ferner sind im Verfahren der Erfindung als Produkt oder Zwischenprodukt erhältliche neue Stoffe Gegenstand von Anspruch 17.

Dadurch, daß als optisch aktive Säure ein N-acyliertes tert-Leucin der allgemeinen Formel VIII eingesetzt wird

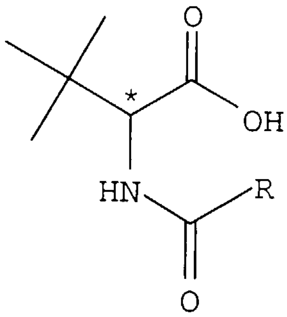

(VIII),

worin R Wasserstoff oder ein gegebenenfalls mit Heteroatomen substituierter oder diese enthaltender Alkyl-, Arylalkyl- oder Arylrest mit bis zu 20 C-Atomen sein kann und * ein Chiralitätszentrum bedeutet, sind optisch aktives tert-Leucinol und seine Derivate nunmehr leicht, sicher, kostengünstig und in hoher Enantiomerenreinheit zugänglich, und zwar sowohl in der (R)- als auch in der (S)-Konfiguration.

Aufgrund des oben Gesagten war es in keinster Weise vorhersehbar oder zu erwarten, daß optisch aktive N-acylierte Derivate des (R)- oder (S)-tert-Leucins, die nach prinzipiell bekannten Verfahren durch Umsetzung von (R)- oder (S)-tert-Leucin mit einem Ester, Säureanhydrid oder Säurechlorid leicht in guter Ausbeute hergestellt werden können, mit tert-Leucinol Salze bilden, in denen eines der beiden Enantiomere mehr oder weniger stark angereichert ist. Zu den in der Erfindung mit Erfolg einsetzbaren Verbindungen der Formel VIII gehören beispielsweise N-Formyl-, N-Benzoyl- und N-(2-Naphthoyl)-tert-Leucin. Von besonderem Vorteil für das erfindungsgemäße Verfahren sind hierbei vor allem die (S)-Enantiomeren.

In erfindungsgemäß bevorzugter Ausführungsform wird als optisch aktive Säure ein optisch aktives N-Formyl-tert-leucin eingesetzt.

So wurde mit N-Formyl-(S)-tert-leucin aus Isopropanol in 45 % Ausbeute ein Salz mit einem Verhältnis R/S-tert-Leucinol von 93,9:6,1 isoliert. Umkristallisation verbesserte die Reinheit auf 98,6:1,4.

Vorteilhafterweise bildeten im Rahmen der Erfindung optisch aktive tert-Leucinderivate, bevorzugt (S)-tert-Leucinderivate, die am Stickstoff mit größeren Aroylresten geschützt waren, bei der Umsetzung mit (RS)-tert-Leucinol Salze, in denen eine optisch aktive Form überwog. Für den Fall, daß (S)-tert-Leucinderivate verwendet wurden, überwog in den untersuchten Beispielen das (R)-tert-Leucinol.

Ganz besonders vorteilhaft kann die Racematspaltung von (RS)-tert-Leucinol mit N-(2-Naphthoyl)-(R)- oder -(S)-tert-leucin durchgeführt werden.

Beim erfindungsgemäßen Verfahren wird also racemisches (RS)-tert-Leucinol durch Umsetzung mit optisch aktiven Säuren in die Enantiomere gespalten, wobei diese zunächst in Form von Salzen mit den optisch aktiven Säuren erhalten werden. Aus diesen Salzen kann in prinzipiell bekannter Weise das optisch aktive tert-Leucinol (Formel I) freigesetzt werden, das entweder in reiner Form isoliert oder in situ zu weiteren Derivaten wie den oben beschriebenen Verbindungen II - VII umgesetzt werden kann. Beide Enantiomere, sowohl (S)- als auch insbesondere das bisher nur sehr schwer zugängliche (R)-tert-Leucinol, sowie deren Derivate sind nach diesem Verfahren leicht und in hoher optischer Reinheit erhältlich.

Die Umsetzung des racemischen (RS)-tert-Leucinols mit der Verbindung der allgemeinen Formel VIII wird bevorzugt in einem organischen Lösungsmittel durchgeführt. Hierfür kommen grundsätzlich die dem Fachmann geläufigen in der Racematspaltung nützlichen Lösungsmittel, wie Alkohole, Ester, Ether, Ketone oder Kohlenwasserstoffe, in Frage. Von besonderem Vorteil sind Alkohole mit 1 - 6 C-Atomen, wie Methanol, Ethanol, Propanol, Isopropanol, n-Butanol oder Isobutanol. Ganz besonders günstig erwies sich die Verwendung von Isopropanol.

Dabei geht man bevorzugt so vor, daß man das racemische (RS)-tert-Leucinol zusammen mit dem Derivat der optisch aktiven Säure im Lösungsmittel unter Erwärmen löst, wobei bevorzugt nur 0,5 Equivalente der optisch aktiven Säure, bezogen auf den racemischen Aminoalkohol, eingesetzt werden. Anschließend wird unter Rühren abgekühlt, wobei ein Salz des Diastereomerensalzpaares zunächst auskristallisiert und das andere Enantiomere des tert-Leucinols in der Mutterlauge angereichert zurückbleibt.

Grundsätzlich kann das erwünschte optisch aktive tert-Leucinol aus dem auskristallisierten Salz auf jede dem Fachmann geläufige Weise isoliert werden. In bevorzugter Ausführungsform der Erfindung wird das optisch aktive tert-Leucinol aus einem Salz mit der optisch aktiven Säure abgetrennt, indem das Salz mit einer wäßrigen Lösung einer starken Säure umgesetzt wird und die optisch aktive Säure durch Abfiltrieren oder durch Extraktion mit einem organischen Lösungsmittel zurückgewonnen wird, danach die wäßrige Lösung basisch gestellt wird und das optisch aktive tert-Leucinol mit einem organischen Lösungsmittel extrahiert und gegebenenfalls durch Einengen, Destillation, Chromatographie oder Umkristallisation eines sauren Salzes isoliert und weiter gereinigt wird.

Hierbei wird also durch Zugabe einer starken Säure, wie beispielsweise Salz- oder Schwefelsäure, die optisch aktive Säure aus dem Salz freigesetzt und geeignet abgetrennt, während das optisch aktive tert-Leucinol nach Abtrennung der optisch aktiven Säure aus der anschließend basisch gestellten wässrigen Lösung auf geeignete Weise isoliert wird. Hierdurch wird in vorteilhafter Weise auch die optisch aktive Säure wiedergewonnen.

Als Extraktionsmittel für optisch aktives tert-Leucinol sind erfindungsgemäß aromatische Kohlenwasserstoffe bevorzugt. Hierzu gehören u. a. Toluol, Xylole und Cumol. Besonders bevorzugt ist Toluol. Andere mit Wasser nicht mischbare organische Lösungsmittel können jedoch ebenfalls eingesetzt werden.

Im Rahmen der Erfindung kann der optische Antipode zu der mit der oben geschilderten Methode erhältlichen optisch aktiven tert-Leucinol-Verbindung besonders günstig aus der Mutterlauge der Kristallisation erhalten werden. Da die Mutterlauge durch Abtrennung eines Salzes des einen Enantiomeren an diesem stark abgereichert ist, ist das in ihr verbliebene und der Rückgewinnung zugängliche tert-Leucinol an dem anderen Enantiomeren stark angereichert. Aus dem zurückgewonnenen tert-Leucinol ist dieses Enantiomere durch Umsetzung mit einer geeigneten optisch aktiven Säure in besonders hoher optischer Reinheit zugänglich.

Beispielsweise kann in der Erfindung die Racematspaltung ganz vorteilhaft mit N-(2-Naphthoyl)-(S)-tert-leucin durch Umsetzung in einem Lösungsmittel, bevorzugt einem Alkohol, besonders bevorzugt Isopropanol, durchgeführt werden. Dafür werden das racemische (RS)-tert-Leucinol sowie das N-(2-Naphthoyl)-(S)-tert-leucin in dem Alkohol unter Erwärmen gelöst. Anschließend wird unter Rühren kontrolliert abgekühlt, wobei das Salz aus (R)-tert-Leucinol und N-(2-Naphthoyl)-(S)-tert-leucin auskristallisiert. Die Ausbeute nach Umkristallisation betrug für diese bevorzugte erfindungsgemäße Variante beispielsweise 70 % bei einem R/S-tert-Leucinol-Verhältnis von 98,7:1,3. Aus der Mutterlauge dieser Racemattrennung kann dann durch Aufkonzentrieren, Aufnehmen in einer wäßrigen Säure, Abfiltrieren des N-(2-Naphthoyl)-(S)-tert-leucins, Alkalisieren des Filtrats und Extraktion mit einem organischen Lösungsmittel das tert-Leucinol isoliert werden, in welchem nun das (S)-Enantiomere stark angereichert ist. Löst man dieses warm in Isopropanol und setzt es mit (S)-Mandelsäure um, so scheidet sich beim Erkalten ein Salz aus (S)-tert-Leucinol und (S)-Mandelsäure ab, das in einem exemplarischen Versuch nach zweimaliger Umkristallisation ein R/S-Verhältnis von 1,0:99,0 zeigte. Die Ausbeute betrug danach immer noch 63 %, bezogen auf das im ursprünglichen racemischen (RS)-tert-Leucinol vorhandene (S)-tert-Leucinol.

Auf diese Weise sind aus racemischem (RS)-tert-Leucinol Salze sowohl des (S)- als auch insbesondere des bisher nur schwer zugänglichen (R)-tert-Leucinols erhältlich, die eine besonders hohe optische Reinheit aufweisen. Wie bereits erwähnt, kann, wenn erforderlich, aus diesen der Aminoalkohol in freier Form isoliert werden. Dies kann in an sich bekannter Weise dadurch geschehen, daß das Salz in Wasser suspendiert oder gelöst wird und diese Mischung mit einer starken Säure versetzt wird, um die optisch aktive Säure freizusetzen, die dann auskristallisiert oder extrahiert werden kann. Dabei wird beispielsweise das N-(2-Naphthoyl)-(S)-tert-leucin durch Kristallisation in 99 % Ausbeute zurückgewonnen, was die vorteilhafte Eignung dieser Verbindung für eine Racematspaltung besonders unterstreicht. Die wäßrige Phase wird dann alkalisiert, das optisch aktive tert-Leucinol daraus mit einem organischen Lösungsmittel extrahiert und gegebenenfalls durch Destillation weiter gereinigt. So wurde (R)-tert-Leucinol in einer Ausbeute von 55 %, bezogen auf das racemische (RS)-tert-Leucinol, und 78 %, bezogen auf das Salz mit N-(2-Naphthoyl)-(S)-tert-leucin, isoliert, wobei das R/S-tert-Leucinol-Verhältnis mit 98,7:1,3 exakt mit dem für das Salz ermittelten Wert übereinstimmte. Der Drehwert des Produktes übertraf mit -38,1° (c = 2, EtOH) sogar die in der Literatur für (S)-tert-Leucinol angegebenen Werte [+37,24° (c = 1,02, EtOH) (Nishiyama, Sakaguchi, Nakamura, Morihata, Kondo, Itoh, 1989; +37° (c = 1, EtOH) (McKennon, Meyers, Drauz, Schwarm, 1993)], wobei das Vorzeichen natürlich entgegengesetzt ist.

Dieses Verfahren wird im folgenden nochmals schematisch dargestellt:

Schema I

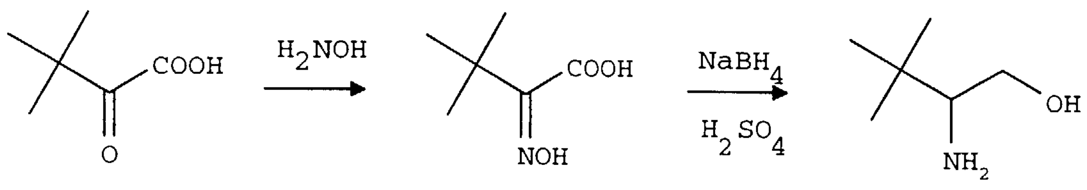


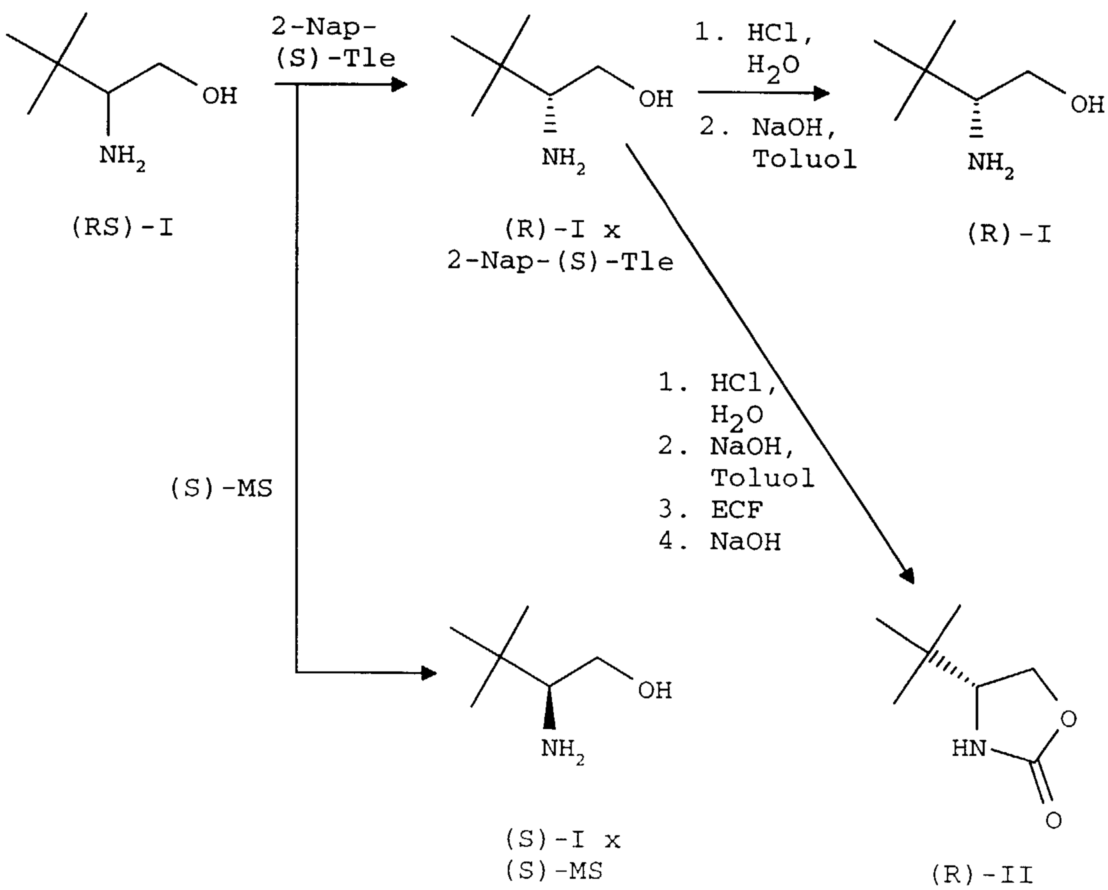


2-Nap-(S)-Tle = N-(2-Naphthoyl)-(S)-tert-leucin
(S)-MS = (S)-Mandelsäure
ECF = Ethylchlorformiat

Das als Ausgangsmaterial benötigte racemische (RS)-tert-Leucinol kann prinzipiell durch Reduktion von (RS)-tert-Leucin nach den für die Reduktion von Aminosäuren bekannten Verfahren hergestellt werden. Überraschenderweise wurde aber gefunden, daß (RS)-tert-Leucinol auch problemlos in guter Ausbeute durch Reduktion des leicht zugänglichen Oxims der Trimethylbrenztraubensäure erhältlich ist. Dieses kann in bekannter, sehr einfacher Weise (Knoop, Landmann, 1914) durch Umsetzung von Trimethylbrenztraubensäure oder einem ihrer Salze mit Hydroxylamin-hydrochlorid in Wasser hergestellt werden und kristallisiert direkt in guter Ausbeute und hoher Reinheit aus der Reaktionslösung aus, während in der zitierten Literaturstelle noch eine aufwendige extraktive Aufarbeitung beschrieben ist.

Die Reduktion des Oxims der Trimethylbrenztraubensäure wird erfindungsgemäß mit einem Alkaliborhydrid, wie Lithium- oder Natriumborhydrid, vorzugsweise dem preiswerten Natriumborhydrid, und einem Aktivator wie Salz- oder Schwefelsäure in einem Lösungsmittel, vorteilhaft einem Ether, insbesondere 1,2-Dimethoxyethan oder Tetrahydrofuran durchgeführt. Diese Reagentien sind für die Reduktion von Aminosäuren bereits bekannt (Abiko, Masamune

1992; McKennon, Meyers, Drauz, Schwarm, 1993). Vorteilhafterweise hat sich herausgestellt, daß sie auch für die Reduktion von Trimethylbrenztraubensäureoxim zu (RS)-tert-Leucinol sehr gut geeignet sind, wobei die Reaktion prinzipiell in einem weiten Temperaturbereich von etwa -20 °C bis zur Siedetemperatur des verwendeten Lösungsmittels, bevorzugt aber so durchzuführen ist, daß die Reagentien etwa bei Raumtemperatur, gegebenenfalls unter Kühlung, zusammengegeben werden und die Umsetzung dann durch Erwärmen auf Temperaturen von bis zu etwa 75 °C vervollständigt wird. Dies war insbesondere aus dem Grund überraschend, weil das reduzierend wirkende Agens offensichtlich in situ erzeugtes Diboran ist (Abiko, Masamune, 1992), an anderer Stelle (H. Feuer, D. M. Braunstein, J. Org. Chem. 1969, 34, 1817) jedoch darauf hingewiesen wurde, daß Oxime durch Diboran erst bei erhöhter Temperatur von 105 - 110 °C in Diglyme-THF zu den korrespondierenden Aminen reduziert werden konnten, während darunter keine Reaktion oder nur Reduktion zu den Hydroxylaminen beobachtet wurde. Für die erfindungsgemäße Reduktion von Trimethylbrenztraubensäureoxim zu (RS)-tert-Leucinol kann dagegen auf derart hohe Temperaturen und den Einsatz des teuren und die Aufarbeitung erschwerenden Lösungsmittels Diglyme verzichtet werden. In einem exemplarischen Versuch wurden so nach Destillation 71 % (RS)-tert-Leucinol erhalten.

Aus dem erfindungsgemäß isolierten optisch aktiven tert-Leucinol können dann in an sich bekannter Weise weitere Derivate des optisch aktiven tert-Leucinols hergestellt werden.

Insgesamt eröffnet das erfindungsgemäße Verfahren einen neuen, besonders einfachen Zugang zu optisch aktivem tert-Leucinol und seinen Derivaten, wobei vorteilhafterweise insbesondere das bisher nur schwer herstellbare (R)-tert-Leucinol und seine Derivate nunmehr leicht erhältlich sind. Es wird durch die folgenden Beispiele weiter erläutert:

Beispiel 1: Herstellung von Trimethylbrenztraubensäureoxim

163 g (1 mol) 93,5 %iges Trimethylbrenztraubensäure-Natriumsalz und 69,5 g (1 mol) Hydroxylaminhydrochlorid wurden bei 40 °C in 450 ml Wasser gelöst. Bei langsamem Abkühlen unter Rühren kristallisierte das Produkt aus. Nach 1,5 h Rühren im Eisbad wurden die Kristalle abfiltriert, mit 150 ml Eiswasser gewaschen und zunächst im Vakuum bei 60 °C vor- und dann im Vakuumexsikkator über Phosphorpentoxid bis zur Gewichtskonstanz nachgetrocknet. Es wurden 117,8 g (Ausbeute 81 %) Trimethylbrenztraubensäureoxim in Form farbloser Kristalle erhalten.
Schmelzbereich: 120-122°C (Zersetzung) [Lit.: 121°C (Knoop, Landmann, 1914)]

| | | | | |
|---|---|---|---|---|
| $C_6H_{11}NO_3$ | ber. | C 49,64 | H 7,64 | N 9,65 |
| (145,16) | gef. | C 49,75 | H 7,89 | N 9,71 |

Beispiel 2: Herstellung von (RS)-tert-Leucinol

Zunächst wurden unter Rühren bei maximal 15 °C zu 480 ml 1,2-Dimethoxyethan (DME) 120 ml (2,25 mol) konz. Schwefelsäure getropft.

Zu einer gerührten Suspension von 171 g (4,5 mol) Natriumborhydrid in 1500 ml DME wurden bei 10 - 30 °C portionsweise 218 g (1,5 mol) Trimethylbrenztraubensäureoxim gegeben, wobei eine heftige Gasentwicklung einsetzte. Anschließend wurde unter Eiskühlung innerhalb von 2,5 h die Schwefelsäure-DME-Lösung zugetropft, wobei die Temperatur von 10 °C auf 40 °C und nach Entfernen der Kühlung auf 55 °C stieg. Danach wurde auf 70 °C erhitzt, abgekühlt und der Ansatz 2 Tage bei Raumtemperatur belassen.

Zur Zerstörung überschüssigen Borhydrids wurden zunächst bei 20 - 55 °C 200 ml Methanol und dann 100 ml Wasser zugetropft, wobei die Temperatur bis 60 °C stieg. Während des gesamten Hydrolysevorgangs wurde heftige Gasentwicklung beobachtet. Anschließend wurde im Vakuum zum dünnen Brei eingedampft und nach Zugabe von weiteren 500 ml Eiswasser das organische Lösungsmittelgemisch abdestilliert. Nach Zusatz von weiteren 600 ml Wasser wurden bei 25 °C 200 ml konz. Salzsäure zugetropft, wobei die Temperatur auf 35 °C stieg und erneut heftige Gasentwicklung einsetzte.

Nach 15 min Nachrühren wurde die Suspension mit 1500 ml Toluol versetzt und mit 300 ml 50 %iger Natronlauge alkalisch gestellt. Die dabei auf 55 °C gestiegene Temperatur wurde weiter auf 70 °C erhöht, worauf die Toluolphase abgetrennt wurde. Die Wasserphase wurde noch zweimal mit je 1 l Toluol bei 70 °C extrahiert. Die vereinigten Toluolphasen wurden danach mit Celite behandelt, filtriert und im Vakuum einrotiert, wobei 158 g eines gelblichen Öls erhalten wurden, das in der Kälte kristallisierte. Destillation lieferte 125,1 g (Ausbeute 71 %) (RS)-tert-Leucinol als farblose Flüssigkeit, die bei Raumtemperatur erstarrte. Ein $^1$H-NMR-Spektrum bestätigte die vorgeschlagene Struktur.

Siedebereich: 86-95 °C / 13 mbar
Schmelzbereich: 34-35°C

| | | | | |
|---|---|---|---|---|
| $C_6H_{15}NO$ | ber. | C 61,49 | H 12,90 | N 11,95 |

(fortgesetzt)

| | | | | |
|---|---|---|---|---|
| (117,19) | gef. | C 61,10 | H 13,15 | N 11,88 |

Beispiel 3: Herstellung von N-(2-Naphthoyl)-(S)-tert-leucin

Zu einer Lösung von 110 g (0,834 mol) (S)-tert-Leucin in 1600 ml Wasser wurde unter Rühren innerhalb von 5 min eine Lösung von 152,4 g (0,80 mol) 2-Naphthoylchlorid in 200 ml THF gegeben, wobei der pH-Wert durch gleichzeitigen Zusatz von 5 M Natronlauge (insgesamt ca. 330 ml) zwischen 7,1 und 7,6 gehalten wurde. Die Temperatur wurde von anfangs 28 °C auf 22 °C gesenkt und der Ansatz insgesamt 1,5 h weiter gerührt, bis sich der pH-Wert nicht mehr änderte. Anschließend wurde das THF im Rotationsverdampfer abdestilliert, die entstandene dünne Kristallsuspension 1 h bei 10 °C gerührt und dann filtriert, um ein auskristallisiertes Nebenprodukt abzutrennen (nach Trocknen 15,4 g).

Das Filtrat wurde dann mit halbkonzentrierter Salzsäure auf pH 2,0 gestellt, worauf sich das Reaktionsprodukt in feinen Kristallen abschied. Die dicke Suspension wurde durch Zugabe von Eis auf etwa 10 °C temperiert, noch 30 min gerührt und filtriert. Nach Waschen mit Wasser und Trocknen im Vakuum bei 50 °C wurden 204 g Feststoff isoliert, der nach DC-Analyse (Diethylether/Methanol = 10/2) jedoch noch ein Nebenprodukt enthielt. Daher wurde das Produkt aus 2500 ml Toluol und nochmals aus 3000 ml Toluol umkristallisiert, worauf nach Trocknen im Vakuum bei 50 °C 169,4 g (Ausbeute 74 %) N-(2-Naphthoyl)-(S)-tert-leucin in Form farbloser Kristalle isoliert wurden. Dünnschichtchromatographisch war das Produkt nunmehr einheitlich, seine Struktur wurde durch ein $^{1}$H-NMR-Spektrum bestätigt. Die Analysen ergaben, daß das so erhaltene Produkt noch 3,12 % Wasser enthielt. Durch schärferes Trocknen (siehe Beispiel 6) konnte die Verbindung dann wasserfrei erhalten werden und dann analytisch charakterisiert werden.

Beispiel 4: Umsetzung von (RS)-tert-Leucinol mit N-(2-Naphthoyl)-(S)-tert-leucin

175,8 g (1,5 mol) (RS)-tert-Leucinol und 214,0 g (0,75 mol) N-(2-Naphthoyl)-(S)-tert-leucin wurden in 4500 ml Isopropanol bei 53 °C gelöst. Es bildeten sich spontan bei dieser Temperatur wieder einige Kristalle. Unter langsamem Rühren wurde innerhalb von 6 h von 53 °C auf 39 °C, dann über Nacht auf 24 °C und dann im Eis/Wasser-Bad auf 15 °C abgekühlt. Die ausgefallenen Kristalle wurden abfiltriert und mit 4 x 150 ml Isopropanol gewaschen. Nach Trocknen wurden 218,3 g Produkt erhalten.

R/S-tert-Leucinol-Verhältnis: R : 93,7 6,3 S (ermittelt durch GC an Lipodex E)

Die Mutterlauge wurde auf 2000 ml aufkonzentriert, auf 45 °C erwärmt und bei 40 °C mit wenig Produkt angeimpft. Der Ansatz wurde über Nacht unter Rühren auf Raumtemperatur, dann im Eis/Wasser-Bad auf 15 °C abgekühlt und weitere 4 h gerührt. Nach Filtration, Waschen mit Isopropanol und Trocknen der Kristalle wurden weitere 21,9 g Produkt isoliert.

R/S-tert-Leucinol-Verhältnis: 88,5 R : 11,5 S (ermittelt durch GC an Lipodex E)

Die beiden Chargen wurden vereinigt und in 4000 ml Isopropanol bei 82 °C gelöst. Nach Animpfen bei 78 °C setzte langsame Kristallisation ein. Der Ansatz wurde unter Rühren über Nacht auf Raumtemperatur und wieder im Eis/Wasser-Bad auf 15 °C abgekühlt. Nach 4 h Rühren bei dieser Temperatur wurden die Kristalle abfiltriert und in 4 Portionen mit insgesamt 300 ml 15 °C kaltem Isopropanol gewaschen. Nach Trocknen im Vakuum bei 50 °C wurden 210,6 g (Ausbeute 70 %) des Salzes aus (R)-tert-Leucinol und N-(2-Naphthoyl)-(S)-tert-leucin erhalten. Ein $^{1}$H-NMR- und ein IR-Spektrum waren im Einklang mit der Struktur des Produktes.

Schmelzbereich: 184-189 °C

$[\alpha]_D^{20}$: +41,6° (c = 1, MeOH)

R/S-tert-Leucinol-Verhältnis: 98,7 R : 1,3 S (ermittelt durch GC an Lipodex E)

| | | | | |
|---|---|---|---|---|
| $C_{23}H_{34}N_2O_4$ | ber. | C 68,63 | H 8,49 | N 6,96 |
| (402,52) | gef. | C 68,48 | H 8,75 | N 7,00 |

Beispiel 5: Umsetzung von angereichertem (S)-tert-Leucinol mit (S)-Mandelsäure

Die Mutterlauge der fraktionierten Kristallisation aus Beispiel 4 wurde im Vakuum zum Öl eingedampft. Nach Aufnehmen in 500 ml Wasser wurde das restliche Isopropanol abdestilliert und auf 500 g eingeengt. Nach Erwärmen auf 55°C wurde mit 6 N Salzsäure auf pH 5,5 gestellt, mit etwas N-(2-Naphthoyl)-(S)-tert-leucin angeimpft und bei 55°C

weiter 6 N Salzsäure bis zu einem pH von 1,8 zugesetzt. Dann wurde unter Rühren auf 18°C abgekühlt und bei pH 1,35 filtriert. Die Kristalle wurden mit Wasser gewaschen und im Vakuum bei 75°C getrocknet. Isoliert wurden 34,0 g NMR-spektroskopisch sauberes (2-Naphthoyl)-(S)-tert-leucin.

Das Filtrat wurde mit Natronlauge auf pH 7,0 gestellt und auf 220 g konzentriert. Mit 45 ml 50 %iger Natronlauge wurde pH 13 eingestellt, nach Zugabe von 300 ml Toluol auf 50-55°C erwärmt, die Wasserphase abgetrennt und bei 55°C nochmals mit 300 ml Toluol extrahiert. Die vereinigten organischen Phasen wurden zu 98,2 g (0,82 mol) rohem, leicht gelblichen tert-Leucinol eingedampft, das stark mit dem (S)-Enantiomeren angereichert war.

Der rohe Aminoalkohol wurde in 3000 ml Isopropanol gelöst, filtriert und auf 60°C erwärmt. Nach Zusatz von 114 g (0,75 mol) (S)-Mandelsäure wurden durch Anreiben erste Kristalle erzeugt. Danach wurde unter Rühren langsam abgekühlt, 4 h bei 15°C gerührt und filtriert. Nach Waschen mit 400 ml Isopropanol bei 15°C und Trocknen der Kristalle bei 50°C im Vakuum wurden 157 g Produkt isoliert.

R/S-tert-Leucinol-Verhältnis: 7,7 R : 92,3 S (ermittelt durch GC an Lipodex E)

Zur Umkristallisation wurde das Produkt in 1560 ml Isopropanol heiß gelöst. Nach Erzeugen erster Kristalle bei 76°C wurde unter Rühren langsam abgekühlt, 4 h bei 15°C gerührt, filtriert, das Produkt mit 300 ml Isopropanol bei 15°C gewaschen und wie oben getrocknet. Isoliert wurden 140,0 g.

R/S-tert-Leucinol-Verhältnis: 3,3 R : 96,7 S (ermittelt durch GC an Lipodex E)

Nochmalige Umkristallisation aus 1500 ml Isopropanol nach gleichem Modus lieferte 127,3 g (Ausbeute 63 %) des Salzes aus (S)-tert-Leucinol und (S)-Mandelsäure in Form farbloser Kristalle. Ein $^1$H-NMR- und ein IR-Spektrum waren im Einklang mit der Struktur des Produktes.

Schmelzbereich: 152-157 °C

$[\alpha]_D^{20}$: +72,6° (c = 1, MeOH)

R/S-tert-Leucinol-Verhältnis: R : 1,0 99,0 S (ermittelt durch GC an Lipodex E)

| | | | | |
|---|---|---|---|---|
| $C_{14}H_{23}NO_4$ | ber. | C 62,43 | H 8,61 | N 5,20 |
| (269,34) | gef. | C 62,62 | H 8,87 | N 5,25 |

Beispiel 6: Isolierung von (R)-tert-Leucinol

201,3 g (0,5 mol) des Salzes aus (R)-tert-Leucinol und N-(2-Naphthoyl)-(S)-tert-leucin wurden in 1300 ml Wasser suspendiert. Tropfenweise wurden 50 ml 37 %ige Salzsäure zugesetzt. Anschließend wurde auf 50°C erwärmt, wobei sich ein pH-Wert von 1,65 einstellte. Es wurde gerührt, bis der pH-Wert 15 min konstant blieb, und dann auf 5°C abgekühlt, wobei sich ein pH-Wert von 1,05 einstellte. Nach 30 min Rühren wurden die Kristalle abfiltriert und mit ca. 1 l Eiswasser gewaschen. Nach Trocknen im Vakuum bei 80°C wurden 141,5 g (Ausbeute 99 %) N-(2-Naphthoyl)-(S)-tert-leucin reisoliert, die $^1$H-NMR-spektroskopisch frei von Verunreinigungen waren.

Schmelzbereich: 161 - 162 °C

$[\alpha]_D^{20}$: +39,7° (c = 1, MeOH)

| | | | | |
|---|---|---|---|---|
| $C_{17}H_{19}NO_3$ | ber. | C 71,56 | H 6,71 | N 4,91 |
| (285,33) | gef. | C 71,32 | H 6,79 | N 5,11 |

Das Filtrat wurde mit 10 M Natronlauge auf pH 7,5 eingestellt, im Rotationsverdampfer auf 146 g eingeengt und exakt in zwei Hälften geteilt. Die eine Hälfte wurde zur Herstellung von (R)-4-tert-Butyl-2-oxazolidinon verwendet (siehe Beispiel 7), die andere auf freies (R)-tert-Leucinol aufgearbeitet.

73 g des genannten Filtrates (enthielt 0,25 mol (R)-tert-Leucinol) wurden weiter auf 60 g konzentriert und nach Zusatz von 100 ml Toluol mit 10 M Natronlauge auf pH 13,0 eingestellt. Nach Erwärmen auf 50°C wurde die Toluolphase abgetrennt und die Wasserphase bei 50°C nochmals mit 75 ml Toluol extrahiert. Die vereinigten organischen Phasen wurden mit Natriumsulfat getrocknet und nach Filtration zu 24,2 g eines fast farblosen Öls eingeengt. Destillation lieferte 22,9 g (Ausbeute 78 %, bezogen auf die Hälfte des eingesetzten Salzes aus (R)-tert-Leucinol und N-(2-Naphthoyl) - (S) -tert-leucin) (R) -tert-Leucinol als farbloses, hygroskopisches Öl, das bei Raumtemperatur erstarrte. Ein $^1$H-NMR-Spektrum bestätigte die Struktur des Produktes.

Siedebereich: 89-91°C / 13 mbar

$[\alpha]_D^{20}$: -38,1° (c = 2, EtOH)

R/S-tert-Leucinol-Verhältnis: 98,7 R : 1,3 S (ermittelt durch GC an Lipodex E)

| | | | | |
|---|---|---|---|---|
| $C_6H_{15}NO$ (117,19) | ber. gef. | C 61,49 C 61,48 | H 12,90 H 13,64 | N 11,95 N 12,07 |

**Patentansprüche**

1. Verfahren zur Herstellung von optisch aktivem tert-Leucinol (Formel I),

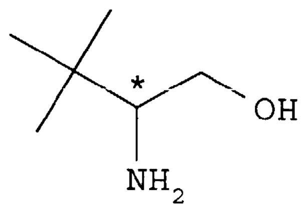

(I)

worin ein * ein Chiralitätszentrum bedeutet, bei welchem Verfahren racemisches (RS)-tert-Leucinol durch Umsetzung mit einer optisch aktiven Säure in ein diastereomeres Salzpaar überführt wird, dieses dann fraktioniert kristallisiert wird und daraus das optisch aktive tert-Leucinol durch Abtrennung der optisch aktiven Säure freigesetzt und ggf. in Substanz isoliert wird,
**dadurch gekennzeichnet**,
daß als optisch aktive Säure ein N-acyliertes tert-Leucin der allgemeinen Formel VIII eingesetzt wird

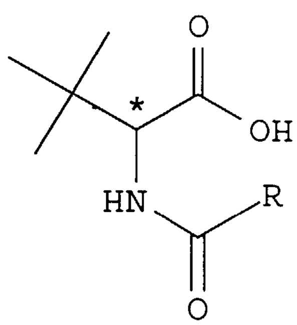

(VIII),

worin R Wasserstoff oder ein gegebenenfalls mit Heteroatomen substituierter oder diese enthaltender Alkyl-, Arylalkyl- oder Arylrest mit bis zu 20 C-Atomen sein kann und * ein Chiralitätszentrum bedeutet.

2. Verfahren nach Anspruch 1,
**dadurch gekennzeichnet**,
daß als optisch aktive Säure ein optisch aktives N-Formyl-tert-leucin eingesetzt wird.

3. Verfahren nach Anspruch 1,
**dadurch gekennzeichnet,**
daß als optisch aktive Säure ein optisch aktives N-(2-Naphthoyl)-tert-leucin eingesetzt wird.

4. Verfahren nach einem der Ansprüche 1 bis 3,
**dadurch gekennzeichnet**,
daß als Lösungsmittel für die fraktionierte Kristallisation ein Alkohol mit 1 - 6 C-Atomen eingesetzt wird.

5. Verfahren nach Anspruch 4,
**dadurch gekennzeichnet,**
daß als Lösungsmittel für die fraktionierte Kristallisation Isopropanol verwendet wird.

**6.** Verfahren nach einem der vorhergehenden Ansprüche,
**dadurch gekennzeichnet**,
daß das optisch aktive tert-Leucinol aus einem Salz mit der optisch aktiven Säure abgetrennt wird, indem das Salz mit einer wäßrigen Lösung einer starken Säure umgesetzt wird und die optisch aktive Säure durch Abfiltrieren oder durch Extraktion mit einem organischen Lösungsmittel zurückgewonnen wird, danach die wäßrige Lösung basisch gestellt wird und das optisch aktive tert-Leucinol mit einem organischen Lösungsmittel extrahiert und gegebenenfalls durch Einengen, Destillation, Chromatographie oder Umkristallisation eines sauren Salzes isoliert und weiter gereinigt wird.

**7.** Verfahren nach Anspruch 6,
**dadurch gekennzeichnet,**
daß als Extraktionsmittel für das optisch aktive tert-Leucinol ein aromatischer Kohlenwasserstoff verwendet wird.

**8.** Verfahren nach Anspruch 7,
**dadurch gekennzeichnet,**
daß als aromatischer Kohlenwasserstoff Toluol eingesetzt wird.

**9.** Verfahren nach einem der Ansprüche 1 und 3 - 8,
**dadurch gekennzeichnet,**
daß das racemische (RS)-tert-Leucinol mit N-(2-Naphthoyl)-(S)-tert-leucin in einem $C_1$-$C_6$-Alkohol umgesetzt wird, das Salz aus (R)-tert-Leucinol und N-(2-Naphthoyl)-(S)-tert-leucin, gegebenenfalls in mehreren Teilmengen, abgetrennt und gegebenenfalls durch Umkristallisation weiter gereinigt wird, die Mutterlauge der fraktionierten Kristallisation eingeengt und in einer wäßrigen sauren Lösung aufgenommen wird, das ausgefallene N-(2-Naphthoyl)-(S)-tert-leucin abfiltriert wird, das Filtrat basisch gestellt wird, das tert-Leucinol, das nun überwiegend das (S)-Enantiomere enthält, mit einem organischen Lösungsmittel extrahiert wird, der Extrakt eingedampft und in einem niederen Alkohol aufgenommen wird und aus dieser Lösung durch Umsetzung mit (S)-Mandelsäure das Salz aus (S)-tert-Leucinol und (S)-Mandelsäure, gegebenenfalls in mehreren Teilmengen, isoliert und gegebenenfalls durch Umkristallisation weiter gereinigt wird.

**10.** Verfahren nach einem der vorhergehenden Ansprüche,
**dadurch gekennzeichnet,**
daß das racemische (RS)-tert-Leucinol durch Reduktion von Trimethylbrenztraubensäureoxim oder einem Ester dieser Substanz hergestellt wird.

**11.** Verfahren nach Anspruch 10,
**dadurch gekennzeichnet,**
daß als Reduktionsmittel Natrium- oder Lithiumborhydrid mit einem Aktivator eingesetzt wird.

**12.** Verfahren nach Anspruch 11,
**dadurch gekennzeichnet,**
daß als Aktivator Chlorwasserstoff oder Schwefelsäure eingesetzt wird.

**13.** Verfahren nach einem der Ansprüche 10 - 12,
**dadurch gekennzeichnet,**
daß die Reaktion in einem Lösungsmittel mit Etherstruktur durchgeführt wird.

**14.** Verfahren nach Anspruch 13,
**dadurch gekennzeichnet,**
daß die Reaktion zwischen -20 °C und der Siedetemperatur des verwendeten Lösungsmittels durchgeführt wird.

**15.** Verfahren nach einem der Ansprüche 10 - 14,
**dadurch gekennzeichnet,**
daß das Reaktionsgemisch nach beendeter Reaktion durch Zugabe von Alkohol, Wasser und gegebenenfalls einer Säure hydrolysiert wird, das organische Lösungsmittel abdestilliert wird, die Wasserphase dann alkalisch gestellt wird, das racemische (RS)-tert-Leucinol dann mit einem organischen Lösungsmittel extrahiert wird und nach Einengen erforderlichenfalls durch Destillation, Chromatographie oder Umkristallisation eines Salzes weiter gereinigt wird.

16. Verfahren nach Anspruch 15,
**dadurch gekennzeichnet,**
daß zur Extraktion des (RS)-tert-Leucinols ein aromatischer Kohlenwasserstoff, bevorzugt Toluol, eingesetzt wird.

17. Nach einem in den Ansprüchen 1 - 16 beschriebenen Verfahren als Produkt oder Zwischenprodukt erhältliche neue Verbindungen: N-(2-Naphthoyl)-(S)-tert-leucin, Salz aus (R)-tert-Leucinol und N-(2-Naphthoyl)-(S)-tert-leucin, Salz aus (S)-tert-Leucinol und (S)-Mandelsäure.

## Claims

1. Process for the production of optically active tert-leucinol (formula I),

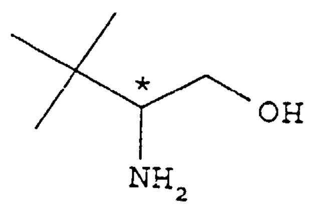

(I)

where a * signifies a chiral centre, in which process racemic (RS)-tert-leucinol is converted to a pair of diastereomeric salts by reaction with an optically active acid, these are then fractionally crystallised and the optically active tert-leucinol is released therefrom by separating off the optically active acid and is optionally isolated in substance, characterised in that
an N-acylated tert-leucine of the general formula VIII is used as the optically active acid

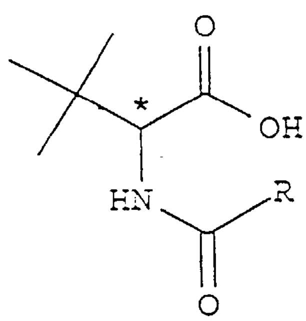

(VIII),

where R signifies hydrogen or an alkyl, arylalkyl or aryl group with up to 20 C atoms, optionally substituted with heteroatoms or containing these, and * signifies a chiral centre.

2. Process according to claim 1,
characterised in that
an optically active N-formyl-tert-leucine is used as the optically active acid.

3. Process according to claim 1,
characterised in that
an optically active N-(2-naphthoyl)-tert-leucine is used as the optically active acid.

4. Process according to one of claims 1 to 3,
characterised in that
an alcohol with 1 - 6 C atoms is used as the solvent for the fractional crystallisation.

5. Process according to claim 4,
characterised in that

isopropanol is used as the solvent for the fractional crystallisation.

**6.** Process according to one of the above claims,
characterised in that
the optically active tert-leucinol is separated off from a salt with the optically active acid in that the salt is reacted with an aqueous solution of a strong acid and the optically active acid is recovered by filtering off or by extraction with an organic solvent, the aqueous solution is then basified and the optically active tert-leucinol is extracted with an organic solvent and optionally isolated by evaporation, distillation, chromatography or recrystallisation of an acidic salt and further purified.

**7.** Process according to claim 6,
characterised in that
an aromatic hydrocarbon is used as the extracting agent for the optically active tert-leucinol.

**8.** Process according to claim 7,
characterised in that
toluene is used as the aromatic hydrocarbon.

**9.** Process according to one of claims 1 and 3 - 8,
characterised in that
the racemic (RS)-tert-leucinol is reacted with N-(2-naphthoyl)-(S)-tert-leucine in a $C_1$-$C_6$ alcohol, the salt of (R)-tert-leucinol and N-(2-naphthoyl)-(S)-tert-leucine is separated off, optionally in several partial quantities, and optionally further purified by recrystallisation, the mother liquor of the fractional crystallisation is concentrated and taken up in an aqueous acidic solution, the precipitated N-(2-naphthoyl)-(S)-tert-leucine is filtered off, the filtrate is basified, the tert-leucinol, which now contains predominantly the (S)-enantiomer, is extracted with an organic solvent, the extract is concentrated by evaporation and taken up in a lower alcohol and the salt of (S)-tert-leucinol and (S)-mandelic acid is isolated from this solution by reaction with (S)-mandelic acid, optionally in several partial quantities, and optionally further purified by recrystallisation.

**10.** Process according to one of the above claims,
characterised in that
the racemic (RS)-tert-leucinol is prepared by reduction of trimethylpyruvic acid oxime or an ester of this substance.

**11.** Process according to claim 10,
characterised in that
sodium or lithium borohydride is used with an activator as the reducing agent.

**12.** Process according to claim 11,
characterised in that
hydrogen chloride or sulphuric acid is used as the activator.

**13.** Process according to one of claims 10 - 12,
characterised in that
the reaction is carried out in a solvent with an ether structure.

**14.** Process according to claim 13,
characterised in that
the reaction is carried out between -20°C and the boiling point of the solvent used.

**15.** Process according to one of claims 10 - 14,
characterised in that
the reaction mixture is hydrolysed on completion of the reaction by adding alcohol, water and optionally an acid, the organic solvent is distilled off, the aqueous phase is then alkalised, the racemic (RS)-tert-leucinol is then extracted with an organic solvent and further purified after concentration if necessary by distillation, chromatography or recrystallisation of a salt.

**16.** Process according to claim 15,
characterised in that

an aromatic hydrocarbon, preferably toluene, is used for extracting the (RS)-tert-leucinol.

**17.** Novel compounds obtainable as a product or intermediate by one of the processes described in claims 1 - 16: N-(2-naphthoyl)-(S)-tert-leucine, salt of (R)-tert-leucinol and N-(2-naphthoyl)-(S)-tert-leucine, salt of (S)-tert-leucinol and (S)-mandelic acid.

## Revendications

**1.** Procédé pour la préparation de tert-leucinol optiquement actif (formule I)

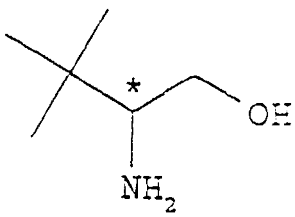

(I)

dans laquelle * désigne un centre de chiralité, procédé dans lequel on transforme du (RS)-tert-leucinol racémique par mise en réaction avec un acide optiquement actif en une paire de sels diastéréoisomère, on soumet cette dernière à une cristallisation fractionnée et on en libère le tert-leucinol optiquement actif par dédoublement de l'acide optiquement actif et on l'isole éventuellement en soi,
caractérisé en ce qu'on met en oeuvre, à titre d'acide optiquement actif, une tert-leucine N-acylée répondant à la formule générale VIII

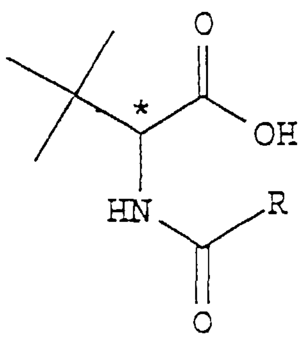

(VIII),

dans laquelle R peut représenter un atome d'hydrogène ou encore un radical alkyle, un radical arylalkyle ou un radical aryle contenant jusqu'à 20 atomes de carbone, le cas échéant portant des hétéroatomes titre de substituants ou contenant ces derniers, et * représente un centre de chiralité.

**2.** Procédé selon la revendication 1,
caractérisé en ce qu'on utilise, à titre d'acide optiquement actif, une N-formyl-tert-leucine optiquement active.

**3.** Procédé selon la revendication 1,
caractérisé en ce qu'on utilise, à titre d'acide optiquement actif, une N-(2-naphtoyl)-tert-leucine optiquement active.

**4.** Procédé selon l'une quelconque des revendications 1 à 3,
caractérisé en ce qu'on met en oeuvre comme solvant pour la cristallisation fractionnée, un alcool contenant de 1 à 6 atomes de carbone.

**5.** Procédé selon la revendication 4,
caractérisé en ce qu'on met en oeuvre comme solvant pour la cristallisation fractionnée, l'isopropanol.

**6.** Procédé selon l'une quelconque des revendications précédentes,
caractérisé en ce qu'on sépare le tert-leucinol optiquement actif à partir d'un sel avec l'acide optiquement actif en

faisant réagir le sel avec une solution aqueuse d'un acide fort et en récupérant l'acide optiquement actif via séparation par filtration ou via extraction dans un solvant organique, ensuite on règle la solution aqueuse pour la rendre basique et on extrait le tert-leucinol optiquement actif dans un solvant organique et, le cas échéant, on l'isole et on le soumet à une purification ultérieure par concentration, distillation, chromatographie ou recristallisation d'un sel acide.

**7.** Procédé selon la revendication 6,
caractérisé en ce qu'on utilise comme agent d'extraction pour le tert-leucinol optiquement actif, un hydrocarbure aromatique.

**8.** Procédé selon la revendication 7,
caractérisé en ce qu'on met en oeuvre comme hydrocarbure aromatique, le toluène.

**9.** Procédé selon l'une quelconque des revendications 1 et 3-8,
caractérisé en ce qu'on fait réagir le (RS)-tert-leucinol racémique avec la N-(2-naphtoyl)-(S)-tert-leucine dans un alcool en $C_1$-$C_6$, on sépare le sel constitué par le (R)-tert-leucinol et par la N-(2-naphtoyl)-(S)-tert-leucine, éventuellement en plusieurs fractions et, le cas échéant, on le soumet à une purification ultérieure par recristallisation, on concentre la liqueur-mère de la cristallisation fractionnée et on la reprend dans une solution acide aqueuse, on sépare par filtration la N-(2-naphtoyl)-(S)-tert-leucine précipitée, on règle le filtrat pour le rendre basique, on extrait dans un solvant organique le tert-leucinol qui contient maintenant principalement l'énantiomère (S), on évapore l'extrait et on le reprend dans un alcool inférieur et, à partir de cette solution, par mise en réaction avec de l'acide (S)-mandélique, on isole le sel constitué par le (S)-tert-leucinol et par l'acide (S)-mandélique, éventuellement en plusieurs fractions et, le cas échéant, on le soumet à une purification ultérieure par recristallisation.

**10.** Procédé selon l'une quelconque des revendications précédentes,
caractérisé en ce qu'on prépare le (RS)-tert-leucinol racémique par réduction de l'oxime de l'acide triméthylpyruvique ou d'un ester de cette substance.

**11.** Procédé selon la revendication 10,
caractérisé en ce qu'on met en oeuvre, à titre d'agent de réduction, du borohydrure de sodium ou de lithium avec un activateur.

**12.** Procédé selon la revendication 11,
caractérisé en ce qu'on met en oeuvre, à titre d'activateur, l'acide chlorhydrique ou l'acide sulfurique.

**13.** Procédé selon l'une quelconque des revendications 10-12,
caractérisé en ce qu'on effectue la réaction dans un solvant possédant une structure éther.

**14.** Procédé selon la revendication 13,
caractérisé en ce qu'on effectue la réaction entre -20°C et la température d'ébullition du solvant utilisé.

**15.** Procédé selon l'une quelconque des revendications 10-14,
caractérisé en ce qu'on hydrolyse le mélange réactionnel au terme de la réaction par addition d'alcool, d'eau et, le cas échéant, d'un acide, on sépare le solvant organique par distillation, on rend ensuite la phase aqueuse alcaline, puis on extrait le (RS)-tert-leucinol racémique dans un solvant organique et, après concentration, en cas de nécessité, on le soumet à une purification ultérieure par distillation, par chromatographie ou par recristallisation d'un sel.

**16.** Procédé selon la revendication 15,
caractérisé en ce que, pour l'extraction du (RS)-tert-leucinol, on met en oeuvre un hydrocarbure aromatique, de préférence le toluène.

**17.** Nouveaux composés que l'on obtient sous forme de produit ou de produit intermédiaire conformément à un procédé décrit dans les revendications 1 à 16: la N-(2-naphtoyl)-(S)-tert-leucine, un sel constitué par le (R)-tert-leucinol et par la N-(2-naphtoyl)-(S)-tert-leucine, un sel constitué par le (S)-tert-leucinol et par l'acide (S)-mandélique.